# FASCICULE DE BREVET EUROPEEN

(11) **EP 0 979 432 B1**
(45) Date de publication et mention de la délivrance du brevet: **18.06.2003**
(21) Numéro de dépôt: 98922885.3
(22) Date de dépôt: 28.04.1998
(51) Int. Cl.: G02B 27/01, A61B 3/00

(54) **SYSTEME OPTIQUE COMBINANT UNE PRESENTATION D'IMAGE ET UNE ANALYSE DE L'OEIL**
OPTISCHES SYSTEM,BESTEHEND AUS EINER BILDDARSTELLUNG UND EINER AUGENANALYSE
OPTICAL SYSTEM COMBINING IMAGE PRESENTATION AND EYE ANALYSIS

(30) Priorité: 29.04.1997 FR 9705256
(43) Date de publication de la demande: 16.02.2000
(73) Titulaire: THALES AVIONICS S.A., 78141 Vélizy Villacoublay Cédex (FR)
(72) Inventeur: GULLI, Christian-Thomson-CSF Propriété Intellect., 94117 Arcueil Cedex (FR); LEGER, Alain-Thomson-CSF Propriété Intellectuelle, 94117 Arcueil Cedex (FR); BIGNOLLES, Laurent-Thomson-CSF Propriété Intellect, 94117 Arcueil Cedex (FR); LAMARQUE, Frédéric-Thomson-CSF Propriété Intellect, 94117 Arcueil Cedex (FR); LE GARGASSON, Jean-François-Thomson-CSF Propriété, 94117 Arcueil Cedex (FR)
(74) Mandataire: Albert, Claude
(86) Numéro de dépôt international: FR9800851
(87) Numéro de publication internationale: WO98049592

(56) Documents cités:
- EP-A- 0 145 563
- EP-A- 0 433 145
- WO-A-93/18428
- FR-A- 2 522 804

## Description

L'invention est relative aux systèmes de présentation d'images. Le but de la présente invention est notamment l'amélioration des systèmes de présentation d'images à un utilisateur pour pouvoir les exploiter dans une large variété de situations différentes.

En particulier, un but de l'invention est d'exploiter des systèmes de présentation d'image dans des situations dans lesquelles un lien doit être établi entre, d'un côté, l'image présentée à l'utilisateur et, d'un autre côté, des paramètres liés à l'utilisateur, et notamment un lien entre l'image présentée et des paramètres de l'oeil de l'utilisateur. Le lien peut exister dans un sens ou dans l'autre, par exemple une modification d'image présentée en fonction de la direction du regard de l'utilisateur, ou réciproquement une analyse de l'oeil pendant la présentation d'une image et en fonction de cette image. Un tel système est connu du document WO93/18428.

Plus précisément, l'invention consiste en un système optique pour présenter une image à un utilisateur, ledit système comprenant une source de production d'éléments de l'image à présenter et une voie optique de transmission de ces éléments d'image entre ladite source et l'oeil de l'utilisateur, caractérisé en ce qu'il comporte également un système d'éclairement du fond de l'oeil et un système de prise d'une image du fond de l'oeil éclairé, le système d'éclairement et le système de prise d'image utilisant au moins une partie de ladite voie optique de transmission.

L'invention peut comporter des moyens de traitement de l'image du fond de l'oeil éclairé, ce qui permet notamment de déterminer la direction du regard de l'utilisateur. La source de production d'éléments de l'image à présenter selon l'invention peut de plus dans ce cas prendre en compte la direction du regard de l'utilisateur. Une telle réalisation de l'invention permet de présenter une image mieux adaptée à la situation d'observation de l'utilisateur.

Un exemple de source de production d'éléments de l'image à présenter est un afficheur comme un dispositif à cristaux liquides ou un tube à rayons cathodiques. Un autre exemple de source est un dispositif d'écriture rétinienne directe, comme par exemple un faisceau laser modulé balayant la rétine. Et ces deux exemples remarquables ne sont pas limitatifs.

La partie de voie optique de transmission, utilisée par le système d'éclairement et le système de prise d'image peut être intégrée dans un dispositif de tête. Ce dispositif est par exemple un appareil mobile comportant une liaison semi-rigide, par exemple une mentonnière, avec l'oeil éclairé ou, de préférence, un casque placé sur la tête de l'utilisateur; l'utilisateur dont on prend une image du fond de l'oeil n'est alors pas contraint à rester immobile devant un dispositif fixe de présentation d'image, son visage est simplement lié à un dispositif de tête permettant la présentation d'image. Une réalisation selon l'invention peut ainsi présenter l'avantage de permettre une observation d'images peu contraignante pour l'utilisateur en laissant à celui-ci une liberté de mouvement de la tête. Et si une telle réalisation comprend un afficheur celui-ci est de préférence monté dans le dispositif de tête (appareil mobile ou casque).

L'invention améliore aussi le confort de l'exploitation du système de présentation d'images dans une réalisation préférée consistant à déporter de la proximité de l'oeil de l'utilisateur une partie du système d'éclairement du fond de l'oeil et une partie du système de prise d'image du fond de l'oeil, à l'aide d'une optique de transport, comprenant par exemple un faisceau de fibres optiques, de façon à autoriser des mouvements de l'utilisateur pendant l'observation de l'image présentée tout en réalisant un dispositif de tête léger.

L'invention peut s'appliquer à la présentation d'image en fonction de la direction du regard de l'observateur, notamment dans un visuel de casque de pilote d'aéronef pour la présentation d'informations d'aide à la navigation et à la visée d'armes.

L'invention peut aussi s'appliquer à l'analyse du mouvement de l'oeil d'un sujet réagissant à une présentation d'images ou à l'analyse du fond de l'oeil du sujet, notamment dans un ophtalmoscope comprenant un dispositif de tête léger autorisant le déplacement de la tête du sujet.

L'invention n'est pas limitée aux applications ci-dessus.

L'invention, ainsi que ses avantages, sera mieux comprise à l'aide de la description suivante faisant référence aux figures annexées lesquelles représentent un arrangement préférentiel mais non limitatif au système selon l'invention.

La figure 1 est un schéma simplifié d'une réalisation de l'invention;

La figure 2 présente un schéma d'un visuel de casque selon l'invention comprenant un dispositif de mesure de la direction du regard de l'utilisateur.

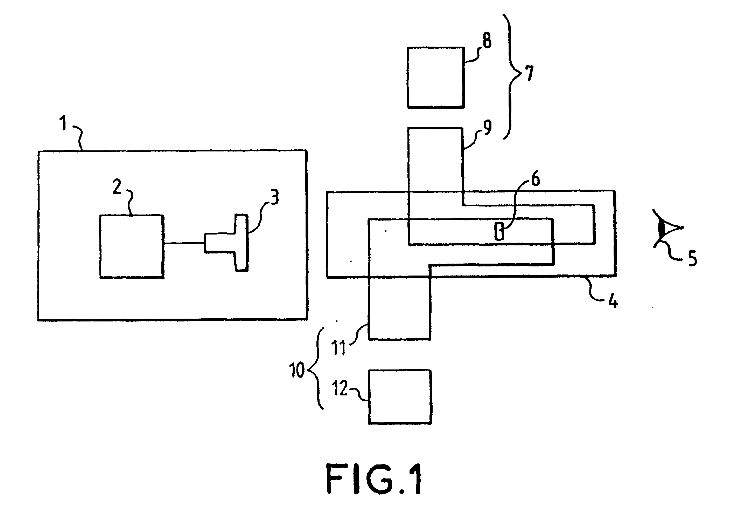

Par un schéma simplifié, la figure 1 illustre une des réalisations possibles de système optique selon l'invention.

Tout système de présentation d'image selon l'invention comprend une source 1 produisant des éléments de l'image à présenter à l'utilisateur.

A titre d'exemple, cette source 1 comprend un générateur d'images 2 alimentant un afficheur classique, tel qu'un imageur à cristaux liquides ou un tube à rayons cathodiques, sur l'écran 3 duquel s'affiche une information lumineuse.

Il est entendu que la source 1 selon l'invention n'est pas limitée à cet exemple.

Selon l'invention, ladite information lumineuse fournie par la source 1 est transmise par une voie optique 4 de transmission entre ladite source 1 et l'oeil 5 de l'utilisateur. Le plus souvent, la voie optique 4 de transmission réalise de plus une transformation, comme par exemple un agrandissement, une collimation ou une correction d'aberrations, de ladite information lumineuse.

La voie optique 4 de transmission permet à l'oeil 5 de l'utilisateur du système optique selon l'invention de percevoir une image à observer.

La voie optique 4 de transmission comprend une optique 6. Cette dernière est, par exemple, une optique de collimation dans le plan focal de laquelle est situé l'écran 3 de l'afficheur de façon à projeter à l'infini l'information lumineuse formée sur l'écran 3 ; et la voie optique 4 permet alors à l'oeil 5 de l'utilisateur de percevoir une information semblant provenir de l'infini.

La voie optique 4 de transmission peut tout aussi bien comprendre une optique 6 réalisant une focalisation à une distance finie ; l'utilisateur perçoit dans ce cas une image qui lui semble située à une distance finie de son oeil 5.

L'invention comprend également un système 7 d'éclairement qui est représenté sur la figure 1 et qui comprend une source 8 d'éclairement et une optique 9 de transmission entre ladite source 8 et l'oeil 5. Le système 7 permet d'éclairer le fond de l'oeil 5 de l'utilisateur.

La partie éclairée est le fond naturel de l'oeil.

Ledit système d'éclairement 7 fonctionne de préférence en lumière non visible ce qui présente l'avantage de ne pas perturber l'utilisateur lors de son observation des images présentées à partir de la source 1.

Ledit système 7 d'éclairement utilise une partie de la voie optique de transmission 4 précédemment décrite.

En plus de l'éclairement du fond de l'oeil 5 de l'utilisateur par le système 7, l'invention comprend un système 10 de prise d'une image de ce fond d'oeil 5.

Dans le système 10, une optique 11 assure la transmission d'au moins une partie de l'onde lumineuse réfléchie par la partie éclairée de l'oeil 5, et se propageant entre l'oeil 5 et un dispositif 12 de réalisation d'une image de l'oeil.

Ledit système 10 de prise d'image utilise une partie de la voie optique de transmission 4 précédemment décrite.

L'invention permet donc de proposer un système de présentation d'image foumissant de plus une image du fond de l'oeil de son utilisateur. L'image est de préférence conforme au fond naturel de l'oeil.

On va maintenant décrire l'invention dans un cas particulier particulièrement intéressant où l'invention est appliquée à un visuel de casque pour pilote d'aéronefs.

De manière générale, un visuel de casque est un dispositif permettant de présenter des informations au porteur du casque.

Et lorsque le porteur du casque est un pilote d'aéronef, les informations sont le plus souvent superposées à la vue du paysage afin de compléter ou de remplacer la vue directe du paysage à travers la visière du casque, ceci sans contraindre le pilote à détourner son regard vers une zone d'affichage du poste de pilotage. Une réalisation connue de visuel de casque consiste à projeter une image sur un combineur devant les yeux de l'utilisateur. Le combineur, qui peut être constitué par la visière du casque, est une surface ayant subi un traitement pour la rendre semi-réfléchissante de telle façon que l'image projetée soit réfléchie vers l'oeil du porteur du casque et qu'en même temps les rayons lumineux émis par le paysage soient aussi transmis par le combineur à l'oeil du porteur du casque.

Le pilote perçoit une image superposée à la vue du paysage. Les informations de l'image présentée sont par exemple des paramètres de pilotage, ou des informations d'aide à la navigation ou à la visée d'une arme.

Certaines informations sont présentées sous forme de caractères alphanumériques, d'autres sous forme de symboles.

Des informations comme l'indication de l'altitude ou de la vitesse de l'aéronef peuvent être présentées à une position fixe par rapport au visage du porteur du casque, tandis que d'autres informations, comme par exemple le tracé d'une piste d'atterrissage, doivent présenter une conformité par rapport au paysage observé et l'image présentée dans ce cas est alors différente selon la direction d'observation du pilote portant le casque.

Cette direction d'observation peut être estimée par une mesure de la direction de l'aéronef et une mesure de la position relative du casque par rapport à cet aéronef. Il existe des casques munis de détecteurs magnétiques de position et dont l'image présentée au porteur d'un tel casque est recalculée en fonction des mesures de position fournies au générateur d'images dudit casque.

Par la mesure de la positon du casque, une telle réalisation connue obtient une assez bonne estimation de l'orientation du visage du porteur du casque ; elle permet ainsi une adaptation des informations présentées pour un porteur de casque dont le point de vue n'est modifié que par le déplacement de la tête c'est-à-dire pour un porteur de casque dont l'orientation des yeux est fixe par rapport à son visage.

Une telle réalisation antérieure présente l'inconvénient d'ignorer le mouvement de rotation de l'oeil lors d'un changement de point d'observation . du pilote ; cet inconvénient est pénalisant car, de façon naturelle, la rotation de l'oeil précède le mouvement de l'ensemble de la tête de l'homme lors d'une observation dynamique avec la tête libre.

Avec de telles réalisations antérieures, le pilote doit privilégier le déplacement de sa tête pour obtenir un bon suivi des images, cependant, parce qu'il porte déjà un casque dont le poids n'est pas négligeable, ses mouvements de tête sont justement plus pénibles et moins rapides qu'une rotation de l'oeil.

L'invention permet de pallier cet inconvénient en prenant en compte la direction du regard du pilote.

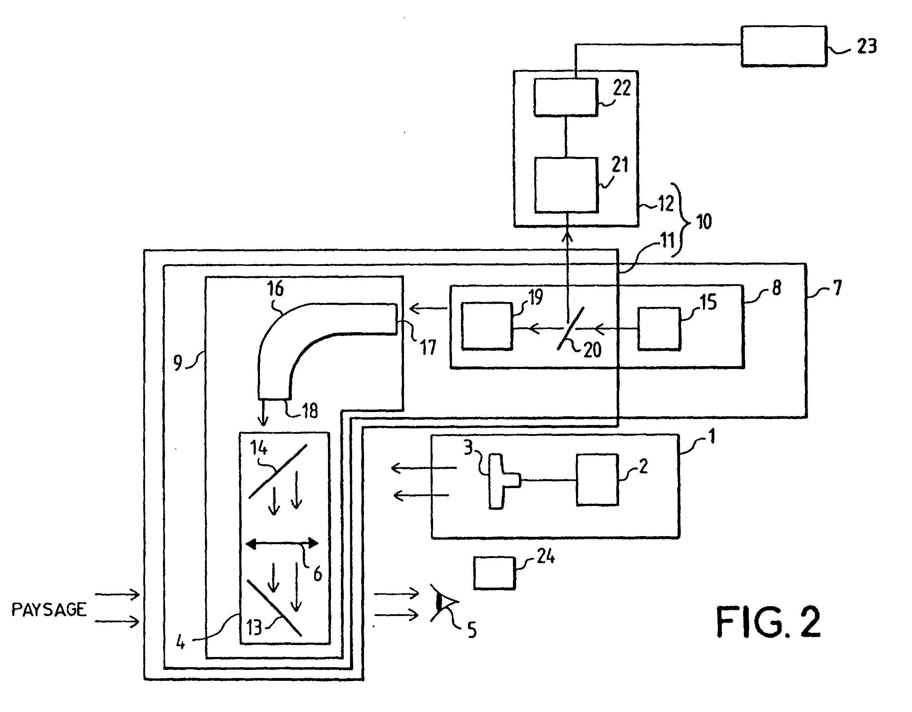

Un visuel selon l'invention, destiné à un casque de pilote d'aéronefs est représenté par un schéma sur la figure 2.

Le visuel de casque comprend, selon l'invention, une source 1 d'éléments d'image à présenter.

Dans la réalisation illustrée par la figure 2, ladite source 1 comprend, de même que celle de l'exemple décrit à l'aide de la figure 1, un générateur 2 d'images alimentant un moyen de visualisation sur l'écran 3 duquel sont affichées des informations en lumière visible.

Le moyen de visualisation est par exemple un élément de la liste non limitative suivante : tube cathodique, imageur à cristaux liquides (à matrice active ou non). Dans cet exemple, on a choisi un tube cathodique.

Le tube cathodique est ici monté dans un casque non représenté sur la figure 2 et porté par le pilote de l'aéronef qui utilise le visuel selon l'invention.

L'oeil 5 de l'utilisateur du visuel de casque est représenté sur la figure 2 ; et il perçoit une image issue de la source 1 par l'intermédiaire d'une voie optique de transmission 4.

La voie optique de transmission 4 comprend une optique 6 de collimation dont le positionnement est tel qu'une image affichée sur l'écran 3 est projetée à l'infini.

La voie optique de transmission 4 comprend également un combineur 13 formé par exemple par une partie de la visière du casque.

L'oeil 5 de l'utilisateur peut donc observer le paysage directement à travers la visière et percevoir en superposition une image en lumière visible renvoyée par le combineur en direction de l'oeil 5.

Par ailleurs, la pupille instrumentale d'un système optique est la zone de l'espace dans laquelle l'oeil de l'utilisateur de ce système optique doit être placé pour permettre une observation avec ledit système.

Comme dans toute utilisation d'un système optique, la perception correcte de l'écran 3 par l'utilisateur de l'invention nécessite le positionnement de son oeil 1 dans la pupille instrumentale de la voie optique de transmission 4. Ici la pupille de l'oeil 1 est dans le plan de la pupille instrumentale de la voie optique 4.

La voie optique de transmission 4 est une optique complexe: son poids et ses dimensions sont suffisamment réduits pour qu'elle soit intégrée au casque, elle comprend des lentilles de corrections d'aberrations et elle présente de plus une pupille étendue.

La pupille instrumentale de la voie optique 4 présente par exemple un diamètre deux à trois fois plus important que le diamètre de la pupille de l'oeil 5. Typiquement ces diamètres sont respectivement d'environ quinze et cinq millimètres.

Dans la réalisation illustrée par la figure 2, la voie optique de transmission 4 comprend également un miroir 14 de renvoi de l'image formée sur l'écran 3 du tube cathodique, le renvoi s'effectuant en direction de l'axe optique de l'optique 6 de collimation.

Les optiques de la voie optique 4, ainsi que le tube cathodique sont ainsi placés dans le casque de façon à obtenir une répartition de poids sur la tête du pilote compatible avec les conditions extrêmes d'utilisation du casque comme, par exemple, l'éjection du pilote hors de l'aéronef.

Selon l'invention, le visuel de casque comprend un système 7 d'éclairement du fond de l'oeil 5 de l'utilisateur. La partie éclairée est le fond réel de l'oeil.

Sur la figure 2, la source d'éclairement 8 du système 7 d'éclairement éclaire quelques fibres d'une première extrémité 17 d'un faisceau 16 de fibres optiques. L'onde lumineuse d'éclairement se propage le long de ces fibres optiques jusqu'à la seconde extrémité 18 du faisceau 16 de fibres, puis elle se propage, par l'intermédiaire de la voie optique de transmission 4 déjà décrite, et de la pupille de l'oeil 5 jusqu'à la rétine de l'oeil 5 de l'utilisateur.

Dans le système 7 d'éclairement de cette réalisation particulière de l'invention, l'optique 9 de transmission entre ladite source 8 et l'oeil 5 de l'utilisateur comprend le faisceau 16 de fibres optiques et la voie optique de transmission 4.

La seconde extrémité 18 du faisceau 16 de fibres optiques est sensiblement plane et est située dans le plan focal de l'optique de collimation 6 déjà décrite.

L'éclairement d'un groupe A de fibres optiques sur la première extrémité 17 du faisceau 16 conduit à l'éclairement d'une zone B de la rétine de l'oeil 5.

Du fait de la collimation, la zone B correspondant à un groupe A déterminé reste fixe si l'oeil 5 se déplace selon des mouvements de translation à l'exclusion de tout mouvement de rotation, tout en restant dans la pupille instrumentale de la voie optique de transmission 4.

La source 8 du système d'éclairement 7 comprend de plus un système de balayage 19 assurant l'éclairement successif de différents groupes A de fibres optiques sur la première extrémité 17 du faisceau 16 de fibres de façon à balayer la rétine par la zone éclairée B. Le balayage est de préférence de type vidéo.

Le système de balayage 19 comprend par exemple un balayage mécanique réalisé par un miroir galvanométrique.

Dans la réalisation de l'invention illustrée par la figure 2, le faisceau 16 de fibres optiques permet de déporter la source d'éclairement 8 à l'extérieur du casque et à distance de celui-ci par connecteur largable.

La source d'éclairement 8 est de préférence une source présentant une longueur d'onde limitée au domaine de la lumière non visible pour ne pas perturber l'utilisateur.

La source 8 comprend par exemple une diode laser infrarouge 15. Cependant la source 8 n'est pas limitée à une source de lumière cohérente. Dans la réalisation ici décrite, la diode laser 15 présente une puissance lumineuse intéressante.

Par rapport aux visuels de casque de l'art antérieur, le visuel de casque illustré par la figure 2 présente l'avantage de posséder une fonction supplémentaire d'éclairement du fond de l'oeil 5 de l'utilisateur sans présenter de supplément de poids important.

Selon l'invention le système de présentation d'image à un utilisateur qu'est le visuel illustré par la figure 2, comprend de plus un système 10 de prise d'une image du fond de l'oeil 5 éclairé. L'image est conforme au vrai fond de l'oeil éclairé.

La zone B éclairée de la rétine renvoie une onde lumineuse qui suit le chemin inverse de l'onde d'éclairement déjà décrite. L'onde émise par la rétine éclairée de l'oeil 5 traverse la voie optique de transmission 4, elle entre dans le faisceau 16 de fibres optiques par quelques fibres de la seconde extrémité 18 de ce faisceau 16, elle se propage le long de ces fibres jusqu'à la première extrémité 17 puis jusqu'au système de balayage 19.

Dans la réalisation ici décrite, le système de balayage 19 n'a pas changé de position de balayage pendant le bref instant correspondant au parcours aller et retour de la lumière entre le système de balayage 19 et la rétine de l'oeil 5.

A l'issue du système de balayage 19, la lumière renvoyée par la zone éclairée de la rétine de l'oeil 5 est sensiblement parallèle à celle émise par la diode laser infrarouge 15 et elle est ensuite réfléchie par un miroir semi-réfléchissant 20 en direction d'un dispositif 12 pour réaliser une image de la rétine de l'oeil 5. L'image est une représentation conforme de la rétine réelle éclairée.

Le miroir semi-réfléchissant 20 réfléchit beaucoup plus qu'il ne transmet, son couple de coefficients caractéristiques est par exemple (0,95/0,05) ou encore (0,9/0,1), il favorise ainsi le flux lumineux de retour en provenance de la rétine de l'oeil 5, lequel est sensiblement inférieur au flux émis par la diode laser 15.

Dans le système 10 de prise d'image selon cette réalisation particulière de l'invention, l'optique 11, dont la définition a été précisée lors de la description de la figure 1, comprend la voie optique de transmission 4, le faisceau 16 de fibres optiques, le système de balayage 19 et le miroir semi-réfléchissant 20.

Ledit dispositif 12 comprend par exemple un détecteur 21 pour réaliser un élément d'image et le détecteur 21 coopère avec un dispositif 22 pour élaborer l'image complète.

Le détecteur 21 détecte l'intensité de la lumière, renvoyée par la zone B éclairée de la rétine de l'oeil 5, par exemple à l'aide d'une photodiode à avalanche. L'intensité détectée dépend de la nature des cellules éclairées du tissu de la rétine.

Le balayage de la rétine de l'oeil 5 par la zone éclairée B permet par détections successives de l'intensité lumineuse émise de réaliser une image de type vidéo de l'ensemble de la surface la rétine de l'oeil 5.

Le faisceau 16 de fibres optiques permet de déporter la partie du système 10 réalisant effectivement l'image de la rétine de l'oeil 5.

L'image du fond de l'oeil 5 présente la vascularisation du tissu de la rétine avec des croisements de vaisseaux sanguins dont la topologie est spécifique de chaque individu et réalise une signature optique. La papille, matérialisant le départ du nerf optique, peut aussi être représentée par le dispositif 12 d'imagerie.

Le visuel de casque de la figure 2 présente ainsi l'avantage de permettre une visualisation du fond de l'oeil de l'utilisateur autorisant par exemple l'analyse de l'état de la rétine, voire l'indication de traitements particuliers de celle-ci, tout en laissant l'utilisateur dont l'oeil est analysé, libre de ses mouvements. Un tel utilisateur ne fixe pas son visage devant un dispositif d'analyse tout aussi fixe, il porte simplement un casque, comprenant le visuel qui vient d'être décrit permettant l'analyse de l'oeil et autorisant des mouvements de tête.

L'image de la rétine de l'oeil 5 qui est fournie par le système 10 de prise d'image présente des caractéristiques particulières.

D'une part, une translation de l'oeil 5 de l'utilisateur par rapport à l'optique de transmission 4 et donc par rapport au casque lui-même ne modifie pas l'image de la rétine de cet oeil fournie par le dispositif 22.

D'autre part, on peut aussi remarquer qu'une rotation de l'oeil 5 conduit à une déformation de l'image de la rétine de cet oeil, et qu'inversement une analyse de la déformation de l'image permet d'estimer le déplacement en rotation de l'oeil par rapport au casque portant le visuel selon l'invention.

Sur la figure 2, on note de plus la présence d'un dispositif 23 assurant un traitement de l'image vidéo fournie par le dispositif 22.

Par traitement d'image, le dispositif 23 analyse quelques points caractéristiques de la rétine comme par exemple la papille et une dizaine de croisements de vaisseaux sanguins. Et l'estimation de la déformation de ces points caractéristiques permet une estimation de la rotation de l'oeil 5.

Le système selon l'invention et illustré par la figure 2 procède par exemple à la prise d'une première image du fond de l'oeil 5 de l'utilisateur et cette image est nommée image de référence.

Plus précisément, cette prise d'image de référence est effectuée par exemple en présentant à l'utilisateur une image particulière avec un réticule et en faisant fixer le regard de l'utilisateur sur le centre de ce réticule.

Ensuite l'utilisateur observe les images présentées par le système de présentation d'images de l'invention, et le dispositif 23 réalise des mesures de déplacement de quelques points caractéristiques de la rétine de l'utilisateur pour estimer la rotation de l'oeil 5 de l'utilisateur par rapport à la direction de référence qui est celle de la direction de fixation du regard lors de la prise de l'image de référence.

Ce traitement présente l'avantage de permettre une estimation d'une rotation quelconque de l'oeil. Cette estimation constitue une mesure de rotation de l'oeil par rapport au casque dans les trois directions de l'espace.

L'invention permet donc la réalisation d'un système comme celui illustré par la figure 2 assurant à la fois une présentation d'image à un utilisateur et une mesure de la direction du regard de cet utilisateur par analyse du fond de l'oeil réel de l'utilisateur: c'est un oculomètre sur fond d'oeil avec une présentation d'images utilisant la même voie optique. II présente l'avantage d'être compact.

Dans une application de visuel de casque, la détermination de la direction du regard permet alors d'agir sur la source 1 d'éléments de l'image à présenter afin de modifier l'image en fonction de cette direction.

Dans d'autres applications, le but peut être de réaliser une observation du fond d'oeil tout en présentant à l'oeil une image dont la structure ou les paramètres induisent sur l'oeil des réactions comme par exemple un changement de direction, qui sont utiles à l'observation effectuée.

Par ailleurs, l'optique de collimation 6 et la pupille étendue de la voie optique de transmission 4 sont telles qu'un simple positionnement sur la tête du pilote du casque portant le système selon l'invention permet la mesure de la rotation de l'oeil par rapport au casque. En particulier, la mesure fournie par le dispositif selon l'invention n'est pas sensible aux petits déplacements latéraux typiquement inférieurs à dix millimètres du casque par rapport à la tête du porteur.

La mesure de la direction du regard relative au casque de l'utilisateur et déjà décrite peut être complétée par une mesure de la position et de l'orientation du casque, par exemple à l'aide d'un dispositif magnétique 24 de mesure de la position et de l'orientation du casque.

Pour un visuel de casque de pilote d'aéronef, l'invention mesure la direction du regard par rapport au casque, la mesure complémentaire de position et d'orientation du casque est par exemple réalisée par rapport au poste de pilotage par le dispositif 24 et en connaissant de plus l'orientation de l'aéronef, on obtient la direction du regard par rapport au paysage, ce qui permet à l'aide du générateur d'images 2 de présenter des éléments d'images conformes au paysage.

Le système complet illustré par la figure 2 présente l'avantage de foumir une estimation précise de la direction du regard sans contraintes draconiennes sur la position du casque par rapport à la tête de l'utilisateur du système.

L'invention permet alors aussi la visée d'une arme dans la direction indiquée par le regard du pilote.

Et à l'aide du dispositif 24, le système illustré par la figure 2 permet l'analyse fine des mouvements couplés de l'oeil et de la tête en réaction à une présentation d'images et en particulier lorsque les images sont superposées à une vue du paysage.

## Revendications

1. Système optique pour présenter une image à un utilisateur comprenant une source (1) de production d'éléments de l'image à présenter, une voie optique (4) de transmission de ces éléments d'image entre ladite source (1) et l'oeil (5) de l'utilisateur, **caractérisé en ce qu'**il comporte un système (7) d'éclairement du fond de l'oeil distinct de ladite source (1) et un système (10) de prise d'une image du fond de l'oeil (5) éclairé pour déterminer des points caractéristiques de la structure de ce fond de l'oeil, le système (7) d'éclairement et le système (10) de prise d'image utilisant au moins une partie de ladite voie optique de transmission.

2. Système optique selon la revendication 1, **caractérisé en ce que** les points caractéristiques sont des croisements de vaisseaux sanguins.

3. Système optique selon l'une des revendications précédentes, **caractérisé en ce qu'**un des points caractéristiques est la papille.

4. Système optique selon l'une des revendications précédentes, **caractérisé en ce qu'**il comporte des moyens de traitement de l'image du fond de l'oeil (5) pour déterminer à l'aide de cette image la direction du regard de l'utilisateur.

5. Système optique selon la revendication 4, **caractérisé en ce que** ladite partie de la voie optique (4) de transmission utilisée par le système (7) d'éclairement et le système (10) de prise d'image du fond de l'oeil (5) est montée dans un casque porté par l'utilisateur ou sur une plate-forme asservie aux mouvements de la tête de l'utilisateur.

6. Système optique selon la revendication 5, **caractérisé en ce que** ladite partie de la voie optique (4) de transmission comporte une optique (6) de collimation permettant de présenter à l'oeil (5) de l'utilisateur une image focalisée à l'infini.

7. Système optique selon la revendication 5, **caractérisé en ce que** ladite partie de la voie optique (4) de transmission comporte une optique (6) de focalisation à une distance finie permettant de présenter à l'oeil (5) de l'utilisateur une image semblant placée à une distance finie.

8. Système optique selon la revendication 6 ou 7, **caractérisé en ce que** ladite source (1) de production d'éléments d'image comprend un afficheur (3) placé dans le plan focal de l'optique (6).

9. Système optique selon la revendication 8, **caractérisé en ce que** ledit afficheur (3) est placé dans ledit casque placé sur la tête de l'utilisateur.

10. Système optique selon l'une des revendications 6 ou 7, **caractérisé en ce que** ladite source (1) de production d'éléments d'image est un faisceau lumineux, en lumière visible, associé à des moyens (19) de balayage permettant une écriture d'image directe sur la rétine de l'oeil (5) de l'utilisateur.

11. Système optique selon l'une des revendications 1 à 10, **caractérisé en ce que** le système (7) d'éclairement du fond de l'oeil (5) comprend une source (15) lumineuse associée à un moyen (19) de balayage.

12. Système optique selon la revendication 11 **caractérisé en ce que** la source lumineuse (15) est une source infrarouge.

13. Système optique selon la revendication 11 ou 12, **caractérisé en ce que** le système (10) de prise d'une image du fond de l'oeil (5) comprend un moyen (22) de détection de l'intensité de la lumière émise par le fond de l'oeil (5) éclairé par ladite source (15) lumineuse.

14. Système optique selon la revendication 13, **caractérisé en ce que** le système (10) de prise d'une image du fond de l'oeil (5) comprend également un moyen de balayage pour la réalisation de ladite image.

15. Système optique selon la revendication 14, **caractérisé en ce que** le moyen (19) de balayage du système (7) d'éclairement et le moyen de balayage du système (10) de prise d'une image sont communs.

16. Système optique selon l'une des revendications précédentes prise en combinaison avec la revendication 5, **caractérisé en ce que** le système (7) d'éclairement du fond de l'oeil et le système (10) de prise d'une image du fond de l'oeil (5) éclairé sont partiellement déportés dudit casque à l'aide d'une optique de transport.

17. Système optique selon la revendication 16, **caractérisé en ce que** ladite optique de transport comprend un faisceau (16) de fibres optiques.

18. Système optique selon l'une des revendications précédentes prise en combinaison avec la revendication 4, **caractérisé en ce que** la source (1) de production d'éléments de l'image à présenter tient compte de la direction du regard de l'utilisateur.

## Patentansprüche

1. Optisches System, um einem Benutzer ein Bild zu präsentieren, mit einer Quelle (1) zur Erzeugung von Elementen des zu präsentierenden Bildes, einem optischen Weg (4) zur Übertragung dieser Bildelemente zwischen der Quelle (1) und dem Auge (5) des Benutzers, **dadurch gekennzeichnet, daß** es ein von der Quelle (1) verschiedenes System (7) zur Beleuchtung des Augenhintergrunds und ein System (10) zur Aufnahme eines Bildes des beleuchteten Hintergrunds des Auges (5) umfaßt, um charakteristische Punkte der Struktur dieses Augenhintergrunds zu bestimmen, wobei das System (7) zur Beleuchtung und das System (10) zur Bildaufnahme mindestens einen Teil des optischen Weges zur Übertragung verwenden.

2. Optisches System nach Anspruch 1, **dadurch gekennzeichnet, daß** die charakteristischen Punkte Kreuzungen von Blutgefäßen sind.

3. Optisches System nach einem der vorangehenden Ansprüche, **dadurch gekennzeichnet, daß** einer der charakteristischen Punkte die Papille ist.

4. Optisches System nach einem der vorangehenden Ansprüche, **dadurch gekennzeichnet, daß** es Mittel zur Bearbeitung des Bildes des Hintergrunds des Auges (5) aufweist, um mit Hilfe dieses Bildes die Blickrichtung des Benutzers zu bestimmen.

5. Optisches System nach Anspruch 4, **dadurch gekennzeichnet, daß** der Teil des optischen Weges (4) zur Übertragung, der vom System (7) zur Beleuchtung und vom System (10) zur Bildaufnahme des Hintergrunds des Auges (5) verwendet wird, in einem Helm, der vom Benutzer getragen wird, oder auf einer Plattform, die den Bewegungen des Kopfs des Benutzers nachgeführt wird, montiert ist.

6. Optisches System nach Anspruch 5, **dadurch gekennzeichnet, daß** der Teil des optischen Weges (4) zur Übertragung eine Kollimationsoptik (6) aufweist, die es ermöglicht, dem Auge (5) des Benutzers ein auf unendlich fokussiertes Bild zu präsentieren.

7. Optisches System nach Anspruch 5, **dadurch gekennzeichnet, daß** der Teil des optischen Weges (4) zur Übertragung eine Optik (6) zur Fokussierung auf einen endlichen Abstand aufweist, die es ermöglicht, dem Auge (5) des Benutzers ein Bild zu präsentieren, das in einem endlichen Abstand angeordnet zu sein scheint.

8. Optisches System nach Anspruch 6 oder 7, **dadurch gekennzeichnet, daß** die Quelle (1) zur Erzeugung von Bildelementen ein Anzeigegerät (3) aufweist, das in der Brennebene der Optik (6) angeordnet ist.

9. Optisches System nach Anspruch 8, **dadurch gekennzeichnet, daß** das Anzeigegerät (3) in dem Helm angeordnet ist, welcher auf dem Kopf des Benutzers sitzt.

10. Optisches System nach einem der Ansprüche 6 oder 7, **dadurch gekennzeichnet, daß** die Quelle (1) zur Erzeugung von Bildelementen ein Strahlenbündel mit sichtbarem Licht ist, das mit Mitteln (19) zum Abtasten verbunden ist, die eine direkte Bilderstellung auf der Netzhaut des Auges (5) des Benutzers ermöglichen.

11. Optisches System nach einem der Ansprüche 1 bis 10, **dadurch gekennzeichnet, daß** das System (7) zur Beleuchtung des Hintergrunds des Auges (5) eine Lichtquelle (15) aufweist, die mit einem Mittel (19) zum Abtasten verbunden ist.

12. Optisches System nach Anspruch 11, **dadurch gekennzeichnet, daß** die Lichtquelle (15) eine Infrarotquelle ist.

13. Optisches System nach Anspruch 11 oder 12, **dadurch gekennzeichnet, daß** das System (10) zur Aufnahme eines Bildes des Hintergrunds des Auges (5) ein Mittel (22) zur Erfassung der Intensität des Lichts, das von dem durch die Lichtquelle (15) beleuchteten Hintergrund des Auges (5) emittiert wird, aufweist.

14. Optisches System nach Anspruch 13, **dadurch gekennzeichnet, daß** das System (10) zur Aufnahme eines Bildes des Hintergrunds des Auges (5) auch ein Mittel zum Abtasten für die Darstellung des Bildes aufweist.

15. Optisches System nach Anspruch 14, **dadurch gekennzeichnet, daß** das Mittel (19) zum Abtasten des Systems (7) zur Beleuchtung und das Mittel zum Abtasten des Systems (10) zur Aufnahme eines Bildes gemeinsam sind.

16. Optisches System nach einem der vorangehenden Ansprüche in Kombination mit Anspruch 5, **dadurch gekennzeichnet, daß** das System (7) zur Beleuchtung des Augenhintergrunds und das System (10) zur Aufnahme eines Bildes des beleuchteten Hintergrunds des Auges (5) mit Hilfe einer Transportoptik teilweise aus dem Helm ausgelagert sind.

17. Optisches System nach Anspruch 16, **dadurch gekennzeichnet, daß** die Transportoptik ein Bündel (16) von optischen Fasern umfaßt.

18. Optisches System nach einem der vorangehenden Ansprüche in Kombination mit Anspruch 4, **dadurch gekennzeichnet, daß** die Quelle (1) zur Erzeugung von Elementen des zu präsentierenden Bildes die Blickrichtung des Benutzers berücksichtigt.

## Claims

1. Optical system for presenting an image to a user, comprising a source (1) for producing image elements to be presented and an optical path (4) for transmitting these image elements between the said source (1) and the user's eye (5), **characterized in that** it comprises a system (7) for illuminating the ocular fundus separate from the said source (1) and a system (10) for acquiring an image of the illuminated ocular fundus (5) in order to determine the characteristic points of the structure of this ocular fundus, the illumination system (7) and the image acquisition system (10) using at least part of the said optical transmission path.

2. Optical system according to Claim 1, **characterized in that** the characteristic points are intersections of blood vessels.

3. Optical system according to one of the preceding claims, **characterized in that** one of the characteristic points is the blind spot.

4. Optical system according to one of the preceding claims, **characterized in that** it comprises means for processing the image of the ocular fundus (5) in order to determine the user's viewing direction using this image.

5. Optical system according to Claim 4, **characterized in that** the said part of the optical transmission path (4) used by the illumination system (7) and the system (10) for acquiring an image of the ocular fundus (5) is mounted in a helmet worn by the user or on a platform whose movements are synchronized with those of the user's head.

6. Optical system according to Claim 5, **characterized in that** the said part of the optical transmission path (4) comprises collimation optics (6) making it possible to present an image focused at infinity to the eye (5) of the user.

7. Optical system according to Claim 5, **characterized in that** the said part of the optical transmission path (4) comprises focusing optics (6) focusing at a finite distance enabling an image appearing to be located at a finite distance to be presented to the eye (5) of the user.

8. Optical system according to Claim 6 or 7, **characterized in that** the said source (1) producing image elements comprises a display (3) placed in the focal plane of the optics (6).

9. Optical system according to Claim 8, **characterized in that** the said display (3) is placed in the said helmet worn on the user's head.

10. Optical system according to either of Claims 6 and 7, **characterized in that** the said source (1) producing image elements is a light beam, in the visible light range, combined with scanning means (19) enabling an image to be directly written onto the retina of the eye (5) of the user.

11. Optical system according to one of Claims 1 to 10, **characterized in that** the system (7) for illuminating the ocular fundus (5) comprises a light source (15) combined with a scanning means (19).

12. Optical system according to Claim 11, **characterized in that** the light source (15) is an infrared source.

13. Optical system according to Claim 11 or 12, **characterized in that** the system (10) for acquiring an image of the ocular fundus (5) comprises a means (22) for detecting the intensity of the light transmitted by the ocular fundus (5) illuminated by the said light source (15).

14. Optical system according to Claim 13, **characterized in that** the system (10) for acquiring an image of the ocular fundus (5) also comprises a scanning means for producing the said image.

15. Optical system according to Claim 14, **characterized in that** the scanning means (19) of the illumination system (7) and the scanning means of the image acquisition system (10) are the same.

16. Optical system according to one of the preceding claims taken in combination with Claim 5, **characterized in that** the system (7) for illuminating the ocular fundus and the system (10) for acquiring an image of the illuminated ocular fundus (5) are in part fitted outside the said helmet by means of transport optics.

17. Optical system according to Claim 16, **characterized in that** the said transport optics comprises a bundle (16) of optical fibres.

18. Optical system according to one of the preceding claims taken in combination with Claim 4, **characterized in that** the source (1) for producing image elements to be presented takes the user's viewing direction into account.
